(19) Europäisches Patentamt
European Patent Office
Office européen des brevets

(11) **EP 1 423 369 B1**

(12) **EUROPEAN PATENT SPECIFICATION**

(45) Date of publication and mention
of the grant of the patent:
**17.07.2013 Bulletin 2013/29**

(21) Application number: **02753297.7**

(22) Date of filing: **23.08.2002**

(51) Int Cl.:
*C07D 223/10* (2006.01)    *C07D 201/16* (2006.01)

(86) International application number:
**PCT/NL2002/000558**

(87) International publication number:
**WO 2003/018562 (06.03.2003 Gazette 2003/10)**

(54) **PROCESS FOR DISTILLING ALKALINE CAPROLACTAM PRODUCT AT REDUCED PRESSURE**

VERFAHREN ZUR DISTILLATION VON ALKALISCHEN CAPROLACTAMPRODUKTEN UNTER VERMINDERTEM DRUCK

PROCEDE DE DISTILLATION DE CAPROLACTAME ALCALIN SOUS PRESSION REDUITE

(84) Designated Contracting States:
**AT BE BG CH CY CZ DE DK EE ES FI FR GB GR IE IT LI LU MC NL PT SE SK TR**

(30) Priority: **27.08.2001 EP 01203217**
**27.08.2001 EP 01203215**
**27.08.2001 EP 01203214**

(43) Date of publication of application:
**02.06.2004 Bulletin 2004/23**

(73) Proprietor: **DSM IP Assets B.V.**
**6411 TE Heerlen (NL)**

(72) Inventors:
• **GROOT ZEVERT, Louise, Annemarie**
**NL-6133 VK Sittard (NL)**

• **BINDELS, Joseph, Johannes, Gerardus**
**NL-6142 AG Ein (NL)**
• **JOOSTEN, Rita, Dimphina**
**NL-6214 AG Maastricht (NL)**
• **LEMMENS, Joannes, Albertus, Wilhelmus**
**NL-6041 HV Roermond (NL)**
• **MOSTERT, Eelco**
**NL-6411 LP Heerlen (NL)**

(74) Representative: **Duffy, James Edwin Marsh**
**DSM Intellectual Property**
**P.O. Box 4**
**6100 AA Echt (NL)**

(56) References cited:
**DD-A- 202 870       DE-A- 3 825 524**
**US-A- 3 944 543      US-A- 4 360 461**
**US-A- 4 457 807      US-A- 5 496 941**

**Description**

The invention relates to a process for purifying caprolactam, which process involves distilling alkaline caprolactam product at reduced pressure.

The production of caprolactam generally involves the preparation of caprolactam product, e.g. by Beckmann rearrangement, followed by purification of the caprolactam product to obtain polymerizable grade caprolactam product. The purification can include distillation of the caprolactam product at reduced pressure in the presence of a base, e.g. alkali hydroxide or an alkali amino caproate. Large ranges are mentioned for the amount of added base. DD-A-202870 describes a process in which caprolactam product is purified by distillation under reduced pressure after addition of 0.05 to 1.0 % of the alkali salt of caproic acid to the caprolactam product to be purified. US-A-3,839,324 describes a process in which caprolactam is subjected to an alkaline distillation in vacuo, wherein sodium hydroxide is present as 0.05 -0.5 percent by weight. US-A-5,496,941 describes a process wherein caprolactam is distilled in the presence of a base, the amount of added base as a rule being chosen from 0.05 to 0.9 mol.% based on caprolactam. US-A-4,457,807 describes a process in which untreated caprolactam containing 0.2 mg of solid NaOH per gram of caprolactam (5 mmol NaOH per kg of caprolactam) is supplied to a rectification column wherein rectification is effected under reduced pressure.

It is now found that the quality of the purified caprolactam, e.g. as expressed by the PAN number, is influenced by the alkalinity of the caprolactam product to be distilled. In particular, the PAN number is found to decrease when the alkalinity of the alkaline caprolactam product is decreased to below 5 meq. per kg of caprolactam. The occurrence of undesired fluctuation in quality, e.g. resulting from the oxidation of caprolactam during distillation, is found to decrease when the alkalinity is increased to above 0 meq. per kg of caprolactam.

Accordingly the invention provides a process for purifying caprolactam, which process involves distilling alkaline caprolactam product at reduced pressure, said alkaline caprolactam product comprising (i) caprolactam, (ii) impurities, and (iii) one or more bases selected from the group consisting of alkali hydroxide and alkali amino caproate, characterized in that the alkalinity of the alkaline caprolactam product is less than 5 meq. (5 milliequivalent) per kg of caprolactam.

As used herein alkalinity refers to the alkalinity at a temperature of 25 °C as determined by titration (after diluting the alkaline caprolactam product with water of pH = 5.7 to obtain a solution containing 15 wt.% caprolactam) with a 0.01 N HCl solution to a pH of 5.7, whereby

$$\text{alkalinity} = \frac{v * t}{a * 0.15} * 1000$$

Where:

$v$ = ml of HCl solution added
$t$ = molarity of HCl solution (=0.01)
$a$ = weight of sample (g)

The process according to the invention results in purified caprolactam having a high quality, in particular a low PAN number (as determined in accordance with ISO DIS 8660-Plastics-Determination of permanganate index of caprolactam-Spectrometric method, revision of first edition (ISO 8660; 1988)). Moreover a low value for the extinction (as determined in accordance with ISO 7059 - caprolactam for industrial use-Determination of absorbance at a wavelength of 290 nm) is obtained.

Preferably, the alkalinity of the alkaline caprolactam product is lower than 4.5 meq. per kg of alkaline caprolactam product, more preferably lower than 4.0 meq. per kg, in particular lower than 3.0 meq. per kg, more in particular lower than 2.0 meq. per kg. This further decreases the PAN number.

Preferably, the alkalinity of the alkaline caprolactam product is higher than 0.05 meq. per kg of alkaline caprolactam product, more preferably higher than 0.10 meq. per kg, in particular higher than 0.15 meq. per kg. Increasing the alkalinity to above these values improves the stability, i.e. the sensitivity to occurrence of undesired fluctuations in quality.

As used herein the values mentioned for the alkalinity and concentrations in the alkaline caprolactam product refer to the values of the alkaline caprolactam product to be distilled. viz. when the alkaline caprolactam product is fed to a distillation zone, i.e. the zone in which the distillation is effected, the mentioned values for alkalinity and other concentrations in the alkaline caprolactam product refer to the values in the alkaline caprolactam-product entering the distillation zone.

The alkaline caprolactam product comprises caprolactam. Typically, the alkaline caprolactam product comprises 95 to 99.9 wt.% of caprolactam, in particular at least 97 wt.% of caprolactam, more in particular at least 98 wt.% of caprolactam (relative to the weight of alkaline caprolactam product).

The impurities may be any organic impurities, e.g. low-boiling organic impurities (having a lower boiling point than caprolactam) and/or high-boiling organic impurities (having a higher boiling point than caprolactam).

The alkaline caprolactam product may include water. Preferably, the alkaline caprolactam product comprises less than 5 wt.%, more preferably less than 3 wt.%, in particular less than 2 wt.%, more in particular less than 1 wt.% (relative to the weight of alkaline caprolactam product). A lower amount of water has the advantage that a reduced pressure is easier to create and maintain during distilling.

The alkaline caprolactam-product-comprises one or more bases selected from the group consisting of alkali hydroxide and alkali amino caproate. Preferably the one or more bases are selected from the group consisting of sodium hydroxide, sodium amino caproate, potassium hydroxide, and potassium amino caproate, more preferably selected from the group consisting of sodium hydroxide and sodium amino caproate. Preferably, at least 75 mol.%, more preferably at least 85 mol.%, in particular at least 95 mol.%, more in particular substantially all of said one or more bases is alkali amino caproate. Increasing the relative amounts of alkali amino caproate has the advantage that the occurrence of polymerization during the distillation is lessened. The formation of oligomers and polymers is disadvantageous since it may result fouling of the distillation equipment. Moreover caprolactam is lost. The above percentages are given relative to the total molar quantity of said one or more bases.

The process according to the invention is preferably a process for the purification of caprolactam product, said caprolactam product comprising (i) caprolactam and (ii) impurities, wherein said process comprises adding one or more of the bases to said caprolactam product to yield the alkaline caprolactam product. Preferably, the alkali hydroxide is added as an aqueous solution of alkali hydroxide.

Typically, the caprolactam product comprises 15 to 99.9 wt.% of caprolactam, in particular at least 50 wt.% of caprolactam, more in particular at least 75 wt.% of caprolactam. Typically, the caprolactam product is aqueous caprolactam product comprising water. Typically the sum quantity of water and caprolactam and water in the caprolactam product is preferably at least 95 wt.%, in particular at least 97 wt.%, more in particular at least 98 wt.%. These percentages are given relative to the weight of the caprolactam product. In one embodiment of the invention, the caprolactam product has an acidity of between 0 and 5 meq. per kg caprolactam, is neutral, or has an alkalinity of between 0 and 5 meq. per kg caprolactam. In this embodiment the alkaline caprolactam product can be prepared by adding very small amounts of said one ore more bases. This results in purified caprolactam having a good quality, as expressed by the PAN number, and a good stability. As used herein acidity refers to the acidity at a temperature of 25 °C as determined by titration (after diluting the alkaline caprolactam product with water of pH = 5.7 to obtain a solution containing 15 wt.% caprolactam) with a 0.01 N NaOH solution to a pH of 5.7, whereby

$$\text{acidity} = \frac{v * t}{a * 0.15} * 1000$$

Where:

   $v$ = ml of NaOH solution added
   $t$ = molarity of NaOH solution (=0.01)
   $a$ = weight of sample (g)

Preferably, the caprolactam product is neutral or has an alkalinity of between 0 and 5 meq. per kg of caprolactam. In an embodiment, the process comprises adding between 0.05 and 10 mmol of the one or more bases per kg caprolactam, preferably between 0.05 and 5.0 mmol per kg, more preferably between 0.10 and 4.5 mmol per kg, in particular between 0.15 and 3.0 mmol per kg, more in particular between 0.20 and 2.0, most preferably less than 1.0 mmol per kg caprolactam. Preferably, the amount of added base is decreased when the caprolactam product is less acidic/more alkaline. In an embodiment, the alkaline caprolactam product is directly obtained after addition of the one or more bases, and the alkaline caprolactam product may be distilled without further steps prior to said distilling. In another embodiment, alkaline product obtained following the addition is subjected to one or more purification steps, e.g. to a step in which water is separated from the alkaline product, to obtain the alkaline caprolactam product to be distilled.

In a preferred embodiment, the process involves adding alkali hydroxide to the caprolactam product, yielding an alkaline product, and reacting at least part of said alkali hydroxide in the alkaline product to form alkali amino caproate prior to the distilling. This has the advantage that the alkali amino caproate can be formed without needing separate equipment for preparing said alkali amino caproate. In this embodiment, the alkali hydroxide is preferably added to caprolactam product comprising at least 3 wt.% water, more preferably at least 5 wt.% of water (relative to the total weight of the caprolactam product). Alkali hydroxide may advantageously be converted to form alkali amino caproate during a water separation step.

The process according to the invention may be a batch process or a continuous process. Preferably, the process is a continuous process.

The caprolactam product to which said one or more bases may be added may be obtained in various ways, e.g. by Beckmann rearrangement. A Beckmann rearrangement of cyclohexanone oxime may be effected in the presence of sulphuric acid or oleum, resulting in a Beckmann rearrangement mixture. A base, preferably ammonia, may be added to the Beckmann rearrangement mixture, resulting in a neutralized Beckmann rearrangement mixture. In an embodiment of the invention the preparation of the caprolactam product includes, (a) recovering from a neutralized Beckmann rearrangement mixture, by extraction with an organic solvent, an organic product comprising the organic solvent and caprolactam, (b) recovering from said organic product, by extraction with water or by evaporation of the organic solvent in the presence of water, an aqueous caprolactam product. Following its recovery from the organic product, the aqueous caprolactam product is preferably hydrogenated in the presence of a hydrogenation catalyst In the event that the aqueous caprolactam product is recovered from the organic product by evaporation of the organic solvent in the presence of water, the organic product is preferably washed with water or with an alkaline aqueous solution prior to said evaporation. In the event that the aqueous caprolactam product is recovered from the organic product by extraction with water, the aqueous caprolactam product is preferably subjected to an ion exchanger prior to hydrogenation. Preferably said one or more bases are added to the aqueous caprolactam product after a hydrogenation step.

The distillation may be carried out in any suitable distillation zone, for instance a distillation column. The distillation is effected at reduced pressure. Preferably the distillation is effected at a pressure of less than 50 kPa, more preferably less than 20 kPa, in particular less than 10 kPa. Preferably, the temperature is between 100 and 200°C, more preferably between 110 and 180 °C. These temperatures refer to the temperature in the bottom of the distillation column in which the distillation is effected. Typically, the distilling includes separating low-boiling organic impurities (having a lower boiling point than caprolactam) from the alkaline caprolactam product and/or separating organic high-boiling impurities (having a higher boiling point than caprolactam) from the alkaline caprolactam product. Preferably, the distilling includes, in a first step, separating out as a top product low-boiling impurities from the alkaline caprolactam product while leaving alkaline caprolactam product containing high-boiling impurities as a bottom product, and, in a second step, separating out high-boiling impurities from the bottom product, and recovering purified caprolactam as a top product.

Preferably, the caprolactam is □-caprolactam

The invention will now be elucidated with reference to the following examples without, however, being limited thereto.

In all examples the specifications given were determined as follows:

PAN: ISO DIS 8660-Plastics-Determination of permanganate index of caprolactam-Spectrometric method, revision of first edition ISO 8660; 1988,

$E_{290}$: ISO 7059 - caprolactam for industrial use- determination of absorbance at a wavelength of 290 nm,

Volatile bases (VB) ISO 8661 - Caprolactam for industrial use - Determination of volatile bases content - Titrimetric method after distillation.

Alkalinity: titration with an aqueous solution of 0.01 M hydrochloric acid.

Example I

In a continuous process for the production of pure caprolactam, a stream of caprolactam product was continuously produced by Beckmann rearrangement of cyclohexanone oxime in the presence of oleum, neutralizing the Beckmann rearrangement mixture with ammonia, separating caprolactam from the neutralized Beckmann rearrangement by extraction techniques. Said stream was subjected to a series of purification steps including purification with an ion exchanger, hydrogenation and a first dewatering. The resulting stream of caprolactam product contained about 85 wt. % caprolactam, about 15 wt.% water, and impurities, and had the following specifications (PAN = 2.6, $E_{290}$ = 0.32, VB = 0.44 meq/kg, alkalinity = 0.02 meq/kg). To this stream was continuously added 4.80 mmol NaOH per kg of caprolactam (as 15% aqueous solution). The resulting stream of alkaline caprolactam product was dewatered in a series of evaporators, the temperatures in the evaporators varying between 80 and 125 °C. The total residence time in and between the evaporators was 3 hours. As a result alkaline caprolactam product was obtained containing about 0.5 wt.% water. In said alkaline caprolactam product at least 90% added base appeared to have been reacted to form sodium amino caproate. The alkaline caprolactam product (alkalinity 4.83 meq/kg) leaving the series of evaporators was distilled in two steps under reduced pressure. In the first step low-boiling impurities-and water-were separated in a distillation-column, at (bottom) temperature of 175 °C, and a pressure of 5.2 kPa, the residence time being several minutes. In the second step high-boiling impurities were separated in a distillation column at a (bottom) temperature of 133 °C, a pressure of 1.2 kPa, the residence time being 1 hour. The specifications of the resulting purified caprolactam are indicated in table 1.

Examples II-VIII

Example I was repeated with the difference that different amounts of NaOH were added, resulting in different values for the alkalinity of the feed of the first distillation step (distillation at 175 °C). The specifications of the caprolactam obtained after distillation are indicated in table 1.

Table 1

| Example | Added NaOH | Alkalinity feed First distillation (175 °C) step | PAN | $E_{290}$ | VB | Alkalinity |
|---------|------------|-----------------------------------|-----|-----------|----|-----------|
| Nr. | mmol NaOH /kg capr | meq OH-/kg | | | meq OH$^-$/kg | meq OH$^-$/kg |
| I | 4.80 | 4.83 | 3.71 | 0.14 | 0.16 | 0.012 |
| II | 2.90 | 2.92 | 3.60 | 0.13 | 0.14 | 0.015 |
| III | 1.25 | 1.29 | 3.54 | 0.13 | 0.11 | 0.017 |
| IV | 0.90 | 0.95 | 2.88 | 0.11 | 0.12 | 0.013 |
| V | 0.75 | 0.78 | 2.89 | 0.12 | 0.18 | 0.012 |
| VI | 0.60 | 0.65 | 2.59 | 0.12 | 0.12 | 0.011 |
| II | 0.50 | 0.55 | 1.15 | 0.06 | 0.11 | 0.024 |
| VIII | 0.30 | 0.32 | 1.26 | 0.07 | 0.17 | 0.023 |

These examples show that the PAN number is decreased without impairing the other properties of the caprolactam, if the amount of added NaOHand, consequently, the alkalinity the alkaline caprolactam product is decreased. Moreover the extinction decreases with decreasing amount of added NaOH.

**Claims**

1. Process for distilling alkaline caprolactam product at reduced pressure, said alkaline caprolactam product comprising (i) caprolactam, (ii) organic impurities, and (iii) one or more bases selected from the group consisting of alkali hydroxide and alkali amino caproate, **characterized in that** the alkalinity of the alkaline caprolactam product is less than 5 meq. per kg of caprolactam.

2. Process according to claim 1, wherein the alkalinity of the alkaline caprolactam product is between 0.10 and 3 meq. per kg of caprolactam.

3. Process according to claim 2, wherein the alkalinity of the alkaline caprolactam product is between 0.15 and 2 meq. per kg of caprolactam.

4. Process according to any one of claims 1 to 3, wherein said one or more bases are selected from the group consisting of sodium hydroxide, sodium amino caproate, potassium hydroxide, potassium amino caproate.

5. Process according to any one of claims 1 to 3, wherein at least 75 mol.% of said one or more bases is alkali amino caproate.

6. Process according to any one of claims 1 to 5, wherein said alkaline caprolactam product comprises at least 95 wt. % of caprolactam.

7. Process according to any one of claims 1 to 6 for the purification of caprolactam product, said caprolactam product comprising (i) caprolactam and (ii) impurities, wherein said process comprises adding one or more bases selected from the group consisting of alkali hydroxide and alkali amino caproate to said caprolactam product in an amount so as to yield the alkaline caprolactam product.

8. Process according to claim 7, wherein the caprolactam product has an acidity of between 0 and 5 meq. per kg of caprolactam, is neutral, or has an alkalinity of between 0 and 5 meq. per kg of caprolactam.

9. Process according to claim 7 or claim 8, wherein the process involves adding said one or more bases to said caprolactam product in an amount of less than 10 mmol per kg caprolactam.

10. Process according to claim 9, wherein the process involves adding to said caprolactam product between 0.10 and 5 mmol of said one or more bases per kg of caprolactam.

11. Process according to claim 10, wherein the process involves adding to said caprolactam product between 0.15 and 3 mmol of said one or more bases per kg of caprolactam.

12. Process according to any one of claims 7 to 11, wherein the caprolactam product comprises at least 15 wt.% of caprolactam.

13. Process according to claim 12, wherein the caprolactam product comprises water and the sum quantity of water and caprolactam in the caprolactam product is at least 95 wt.%.

14. Process according to any one of claims 7 to 13, wherein the process involves adding alkali hydroxide to said caprolactam product yielding an alkaline product, and converting at least part of said alkali hydroxide in the alkaline product to form alkali amino caproate prior to said distilling.

15. Process according to any one of claims 1 to 14, wherein the caprolactam is obtained by a Beckmann rearrangement.

16. Process according to any one of claims 1 to 15, wherein the process involves distilling the alkaline caprolactam product at a temperature between 100 and 200 °C.

17. Process according to any one of claims 1 to 16, wherein the process involves distilling the alkaline caprolactam product at a pressure of less than 10 kPa.

18. Process according to any one of claims 1 to 17, wherein said distilling includes separating out low-boiling impurities from the alkaline caprolactam product and/or separating out high-boiling impurities from the alkaline caprolactam product.

19. Process according to claim 18, wherein said distilling includes, in a first step, separating out as a top product low-boiling impurities from the alkaline caprolactam product while leaving alkaline caprolactam product containing high-boiling impurities as a bottom product, and, in a second step, separating out high-boiling impurities from the bottom product, and recovering caprolactam as a top product.

20. Process according to any one of claims 1 to 19, wherein the process is a continuous process.

**Patentansprüche**

1. Verfahren zum Destillieren von alkalischem Caprolactomprodukt bei vermindertem Druck, wobei das alkalische Caprolactamprodukt (i) Caprolactam, (ii) organische Verunreinigungen und (iii) eine oder mehrere Basen aus der Gruppe bestehend aus Alkalihydroxid und Alkaliaminocaproat umfasst, **dadurch gekennzeichnet, dass** die Alkalinität des alkalischen Caprolactamprodukts weniger als 5 meq pro kg Caprolactam beträgt.

2. Verfahren nach Anspruch 1, bei dem die Alkalinität des alkalischen Caprolactamprodukts zwischen 0,10 und 3 meq pro kg Caprolactam liegt.

3. Verfahren nach Anspruch 2, bei dem die Alkalinität des alkalischen Caprolactamprodukts zwischen 0,15 und 2 meq pro kg Caprolactam liegt.

4. Verfahren nach einem der Ansprüche 1 bis 3, bei dem man die Base bzw. Basen aus der Gruppe bestehend aus Natriumhydroxid, Natriumaminocaproat, Kaliumhydroxid und Kaliumaminocaproat auswählt.

5. Verfahren nach einem der Ansprüche 1 bis 3, bei dem es sich bei mindestens 75 Mol-% der Base bzw. Basen um Alkaliaminocaproat handelt.

**6.** Verfahren nach einem der Ansprüche 1 bis 5, bei dem das alkalische Caprolactamprodukt mindestens 95 Gew.-% Caprolactam umfasst.

**7.** Verfahren nach einem der Ansprüche 1 bis 6 zur Reinigung von Caprolactamprodukt, wobei das Caprolactamprodukt (i) Caprolactam und (ii) Verunreinigungen umfasst, bei dem man dem Caprolactamprodukt eine oder mehrere Basen aus der Gruppe bestehend aus Alkalihydroxid und Alkaliaminocaproat in einer solchen Menge zusetzt, dass sich das alkalische Caprolactamprodukt ergibt.

**8.** Verfahren nach Anspruch 7, bei dem das Caprolactamprodukt eine Acidität zwischen 0 und 5 meq pro kg Caprolactam aufweist, neutral ist oder eine Alkalinität zwischen 0 und 5 meq pro kg Caprolactam aufweist.

**9.** Verfahren nach Anspruch 7 oder Anspruch 8, bei dem man dem Caprolactamprodukt die Base bzw. Basen in einer Menge von weniger als 10 mmol pro kg Caprolactam zusetzt.

**10.** Verfahren nach Anspruch 9, bei dem man dem Caprolactamprodukt zwischen 0,10 und 5 mmol der Base bzw. Basen pro kg Caprolactam zusetzt.

**11.** Verfahren nach Anspruch 10, bei dem man dem Caprolactamprodukt zwischen 0,15 und 3 mmol der Base bzw. Basen pro kg Caprolactam zusetzt.

**12.** Verfahren nach einem der Ansprüche 7 bis 11, bei dem das Caprolactamprodukt mindestens 15 Gew.-% Caprolactam umfasst.

**13.** Verfahren nach Anspruch 12, bei dem das Caprolactamprodukt Wasser umfasst und die Gesamtmenge von Wasser und Caprolactam in dem Caprolactamprodukt mindestens 95 Gew.-% beträgt.

**14.** Verfahren nach einem der Ansprüche 7 bis 13, bei dem man dem Caprolactamprodukt Alkalihydroxid zusetzt, was ein alkalisches Produkt ergibt, und mindestens einen Teil des Alkalihydroxids in dem alkalischen Produkt vor der Destillation in Alkaliaminocaproat umwandelt.

**15.** Verfahren nach einem der Ansprüche 1 bis 14, bei dem das Caprolactam durch eine Beckmann-Umlagerung erhalten wird.

**16.** Verfahren nach einem der Ansprüche 1 bis 15, bei dem man das alkalische Caprolactamprodukt bei einer Temperatur zwischen 100 und 200°C destilliert.

**17.** Verfahren nach einem der Ansprüche 1 bis 16, bei dem man das alkalische Caprolactamprodukt bei einem Druck von weniger als 10 kPa destilliert.

**18.** Verfahren nach einem der Ansprüche 1 bis 17, bei dem das Destillieren das Abtrennen von niedrigsiedenden Verunreinigungen aus dem alkalischen Caprolactamprodukt und/oder das Abtrennen von hochsiedenden Verunreinigungen aus dem alkalischen Caprolactamprodukt einschließt.

**19.** Verfahren nach Anspruch 18, bei dem das Destillieren in einem ersten Schritt das Abtrennen von niedrigsiedenden Verunreinigungen aus dem alkalischen Caprolactamprodukt als Kopfprodukt unter Zurücklassung von hochsiedende Verunreinigungen enthaltendem alkalischem Caprolactamprodukt als Sumpfprodukt und in einem zweiten Schritt das Abtrennen von hochsiedenden Verunreinigungen aus dem Sumpfprodukt und die Gewinnung von Caprolactam als Kopfprodukt einschließt.

**20.** Verfahren nach einem der Ansprüche 1 bis 19, bei dem es sich um ein kontinuierliches Verfahren handelt.

**Revendications**

**1.** Procédé de distillation d'un produit caprolactame alcalin à pression réduite, ledit produit caprolactame alcalin comprenant (i) du caprolactame, (ii) des impuretés organiques et (iii) une ou plusieurs bases choisies dans le groupe constitué par un hydroxyde alcalin et un aminocaproate alcalin, **caractérisé en ce que** l'alcalinité du produit caprolactame alcalin est inférieure à 5 méq. par kg de caprolactame.

**2.** Procédé selon la revendication 1, dans lequel l'alcalinité du produit caprolactame alcalin est comprise entre 0,10 et 3 méq. par kg de caprolactame.

**3.** Procédé selon la revendication 2, dans lequel l'alcalinité du produit caprolactame alcalin est comprise entre 0,15 et 2 méq. par kg de caprolactame.

**4.** Procédé selon l'une quelconque des revendications 1 à 3, dans lequel ladite ou lesdites bases sont choisies dans le groupe constitué par l'hydroxyde de sodium, l'aminocaproate de sodium, l'hydroxyde de potassium, l'aminocaproate de potassium.

**5.** Procédé selon l'une quelconque des revendications 1 à 3, dans lequel au moins 75 % en moles de ladite ou desdites bases est un aminocaproate alcalin.

**6.** Procédé selon l'une quelconque des revendications 1 à 5, dans lequel ledit produit caprolactame alcalin comprend au moins 95 % en poids de caprolactame.

**7.** Procédé selon l'une quelconque des revendications 1 à 6, pour la purification d'un produit caprolactame, ledit produit caprolactame comprenant (i) du caprolactame et (ii) des impuretés, dans lequel ledit procédé comprend l'ajout d'une ou de plusieurs bases choisies dans le groupe constitué par un hydroxyde alcalin et un aminocaproate alcalin audit produit caprolactame en une quantité permettant d'obtenir le produit caprolactame alcalin.

**8.** Procédé selon la revendication 7, dans lequel le produit caprolactame a une acidité comprise entre 0 et 5 méq. par kg de caprolactame, est neutre ou a une alcalinité comprise entre 0 et 5 méq. par kg de caprolactame.

**9.** Procédé selon la revendication 7 ou la revendication 8, dans lequel le procédé comprend l'ajout de ladite ou desdites bases audit produit caprolactame en une quantité inférieure à 10 mmol par kg de caprolactame.

**10.** Procédé selon la revendication 9, dans lequel le procédé comprend l'ajout audit produit caprolactame d'entre 0,10 et 5 mmol de ladite ou desdites bases par kg de caprolactame.

**11.** Procédé selon la revendication 10, dans lequel le procédé comprend l'ajout audit produit caprolactame d'entre 0,15 et 3 mmol de ladite ou desdites bases par kg de caprolactame.

**12.** Procédé selon l'une quelconque des revendications 7 à 11, dans lequel le produit caprolactame comprend au moins 15 % en poids de caprolactame.

**13.** Procédé selon la revendication 12, dans lequel le produit caprolactame comprend de l'eau et la quantité totale d'eau et de caprolactame dans le produit caprolactame est d'au moins 95 % en poids.

**14.** Procédé selon l'une quelconque des revendications 7 à 13, dans lequel le procédé comprend l'ajout d'un hydroxyde alcalin audit produit caprolactame pour obtenir un produit alcalin, et la conversion d'au moins une partie dudit hydroxyde alcalin dans le produit alcalin pour former un aminocaproate alcalin avant ladite distillation.

**15.** Procédé selon l'une quelconque des revendications 1 à 14, dans lequel le caprolactame est obtenu par un réarrangement de Beckmann.

**16.** Procédé selon l'une quelconque des revendications 1 à 15, dans lequel le procédé comprend la distillation du produit caprolactame alcalin à une température comprise entre 100 et 200 °C.

**17.** Procédé selon l'une quelconque des revendications 1 à 16, dans lequel le procédé comprend la distillation du produit caprolactame alcalin à une pression inférieure à 10 kPa.

**18.** Procédé selon l'une quelconque des revendications 1 à 17, dans lequel ladite distillation comprend une séparation d'impuretés de point d'ébullition faible du produit caprolactame alcalin et/ou une séparation d'impuretés de point d'ébullition élevé du produit caprolactame alcalin.

**19.** Procédé selon la revendication 18, dans lequel ladite distillation comprend, lors d'une première étape, une séparation en tant que produit de tête d'impuretés de point d'ébullition faible du produit caprolactame alcalin tout en laissant un produit caprolactame alcalin contenant des impuretés de point d'ébullition élevé en tant que produit de fond et, lors d'une seconde étape, une séparation d'impuretés de point d'ébullition élevé du produit de fond, et la récupération de caprolactame en tant que produit de tête.

20. Procédé selon l'une quelconque des revendications 1 à 19, dans lequel le procédé est un procédé continu.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DD 202870 A
- US 3839324 A
- US 5496941 A
- US 4457807 A